# NOUVEAU FASCICULE DE BREVET EUROPEEN

(11) **EP 0 435 714 B2**
(45) Date de publication et mention de la décision concernant l'opposition: **08.07.1998**
(45) Mention de la délivrance du brevet: 09.08.1995
(21) Numéro de dépôt: 90403429.5
(22) Date de dépôt: 03.12.1990
(51) Int. Cl.: C07C 53/50, C07C 51/60

(54) **Procédé de préparation de chlorures d'acides carboxyliques chlorés**
Verfahren zur Herstellung von chlorierten Carbonsäurechloriden
Process for the preparation of chlorinated carboxylic acid chlorides

(30) Priorité: 06.12.1989 FR 8916094
(43) Date de publication de la demande: 03.07.1991
(73) Titulaire: SOCIETE NATIONALE DES POUDRES ET EXPLOSIFS, F-75181 Paris Cédex 04 (FR)
(72) Inventeur: Gauthier, Patricia, F-91590 La Ferte Alais (FR); Le Moult, Michel, F-31400 Toulouse (FR); Rochelle, Jean-Claude, F-31400 Toulouse (FR); Senet, Jean-Pierre, F-77760 La Chapelle La Reine (FR)
(74) Mandataire: Pech, Bernard

(56) Documents cités:
- EP-A- 413 264
- FR-A- 2 227 255

## Description

### Description pour les Etats contractants suivants : BE, CH, DE, ES, FR, GB, IT, LI

L'invention concerne un procédé de préparation de chlorures d'acides carboxyliques chlorés. Elle concerne plus particulièrement un procédé de préparation de chlorures d'acides carboxyliques aliphatiques chlorés par phosgénation de lactones aliphatiques.

Le brevet FR n° 1 080 261 décrit la préparation de chlorures d'acides carboxyliques chlorés par phosgénation de lactones en particulier de la 4-butyrolactone en présence de pyridine comme catalyseur à une température de 120°C. Mais il est difficile de reproduire le résultats mentionnés, en particulier ceux de l'exemple 2, comme il est indiqué dans la demande de brevet européen n° 253 214.

Dans l'article de D.J. Burton et W.M. Koppes (J. Org. Chem., Vol 40, N° 21, 1975, pp. 3026 à 3031), le chlorure aromatique d'orthochlorométhylbenzoyle a été obtenu à partir de la lactone aromatique correspondante avec un dichlorotriphénylphosphorane en excès mais ce composé est un réactif de laboratoire très cher qui n'est pas stable et est particulièrement sensible à l'hydrolyse. De plus il réagit avec le chlorure d'acide formé et il est préférable de l'utiliser après sa complexation avec un acide de Lewis tel que BF₃, ce qui rend encore plus compliqué le procédé. A part la transformation du phtalide mentionné aucun autre essai n'a été réalisé à partir d'autres lactones.

Selon la demande de brevet européen EP n° 253 214 récemment déposée on effectue la phosgénation de lactones telle que les butyrolactone, valérolactone ou caprolactone en présence d'un sel d'ammonium quaternaire à température élevée et de préférence en présence également d'acide chlorhydrique. Mais les sels d'ammonium quaternaires utilisés comme catalyseurs présentent l'inconvénient d'être plutôt instables à température élevée et perdent donc une partie de leur activité. L'acide chlorhydrique employé en quantité importante est particulièrement corrosif à haute température et nécessite des installations et des précautions particulières. Il se produit des réactions parasites. La lactone polymérise et l'agitation correcte du milieu n'est pas aisée. D'autres impuretés se forment par décomposition. La mise en oeuvre de ce procédé est par conséquent difficile. Plusieurs opérations successives sont nécessaires pour obtenir de bons rendements.

Les chlorures d'acides carboxyliques chlorés sont particulièrement recherchés depuis plusieurs années comme intermédiaires de synthèse pour la fabrication de médicaments et de phytosanitaires, il existe donc un besoin de les obtenir de façon simple et économique ainsi qu'avec de bons rendements. Ces chlorures doivent de plus présenter une bonne stabilité à la lumière et au stockage.

Il a maintenant été trouvé que l'on pouvait préparer des chlorures d'acides carboxyliques chlorés de formule 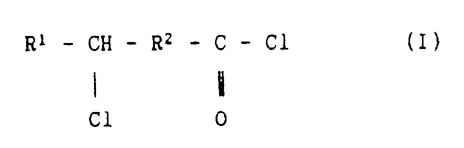 dans laquelle R² représente le radical (CH₂)n, n étant un nombre entier de 2 à 4, ou le radical -CH₂-C(C₆H₅)₂- et
R¹ représente un atome d'hydrogène ou un radical alkyle ayant de 1 à 4 atomes de carbone, par réaction des lactones de formule 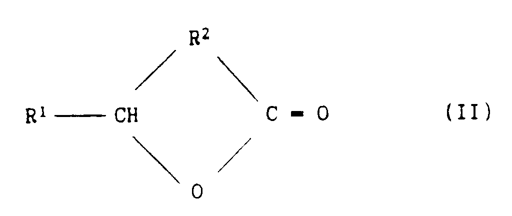 dans laquelle R¹ et R² ont les significations précédentes, avec du phosgène à une température comprise entre 90° et 180°C, en utilisant comme catalyseurs les composés de formule 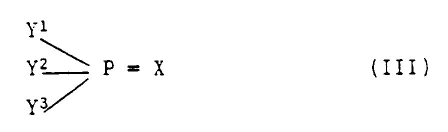 dans laquelle :- Y¹, Y² et Y³, identiques ou différents, représentent chacun un radical phényle qui peut porter un ou plusieurs groupes alkyles ayant jusqu'à 4 atomes de carbone, et X représente un atome d'oxygène ou de soufre,
   - ou bien encore, lorsque R² représente le radical -CH₂-C(C₆H₅)₂-, Y¹, Y² et Y³, identiques ou différents, représentent chacun un radical alkyle linéaire ou ramifié ayant jusqu'à 18 atomes de carbone, et X représente un atome d'oxygène.

On petit également utiliser comme catalyseurs les produits de réaction des composés de formule (III) avec des agents chlorurants, ou encore des mélanges de ces composés.

Les lactones qui peuvent être transformées selon le procédé selon l'invention sont de préférence les lactones de formule II dans laquelle R¹ représente un atome d'hydrogène ou un radical méthyle. A titre d'exemple on peut citer la 4-butyrolactone, la 4-valérolactone, la 5-valérolactone, la 6-caprolactone, la 2,2-diphényl-4-butyrolactone.

Des catalyseurs qui conviennent bien dans le cadre de la présente invention sont les composés de formule III dans lesquels Y¹, Y² et Y³ identiques ou différents représentent un radical alkyle linéaire ou ramifié ayant jusqu' à 12 atomes de carbone ou un radical phényle qui peut porter un ou plusieurs groupes méthyles.

On peut également remplacer les composés de formule III par leurs produits de réaction avec des agents chlorurants connus tels que le chlorure d'oxalyle, le chlorure de thionyle, le phosgène et le pentachlorure de phosphore, qui sont obtenus de façon classique par mélange des composés.

On peut également employer un mélange des composés de formule III et de ces produits de réaction.

Comme exemples de catalyseurs utilisables selon l'invention on peut citer l'oxyde de tributylphosphine, l'oxyde de trioctylphosphine, l'oxyde de tridodécylphosphine, l'oxyde de triphénylphosphine, le sulfure de triphénylphosphine, l'oxyde de tri-p-tolylphosphine.

Les catalyseurs préférés dans le cadre de la présente invention sont l'oxyde de trioctylphosphine et l'oxyde de triphénylphosphine.

Des quantités très faibles des catalyseurs précédemment décrits sont suffisantes pour la bonne réalisation du procédé. Elles sont généralement comprises entre 0,1 et 5% en mole par rapport à la lactone de départ et de préférence entre 0,5 et 2,5%.

La température de réaction peut être comprise entre 90° et 180°C. Elle est de préférence comprise entre 120° et 160°C.

La réaction est de préférence effectuée en absence de solvant. On peut cependant ajouter un milieu solvant inerte vis à vis des composés présents dans le milieu, en particulier du phosgène. Comme solvants on peut citer par exemple les solvants aromatiques chlorés ou non ayant un point d'ébullition suffisamment élevé tels que le monochlorobenzène, le dichlorobenzène, l'isopropylbenzène et les xylènes.

Le phosgène est généralement ajouté en quantité stoechiométrique ou en excès. Cet excès est de préférence compris entre 10 et 20%.

Le procédé selon la présente invention peut être mis en oeuvre soit en discontinu soit en continu à la pression atmosphérique ou à une pression voisine de la pression atmosphérique dans des dispositifs de phosgénation connus.

Selon un mode opératoire approprié, la lactone, le catalyseur et éventuellement le solvant sont tout d'abord introduits dans le réacteur. Le milieu réactionnel est chauffé à la température choisie puis on fait passer progressivement le phosgène gazeux dans le milieu. Le dioxyde de carbone formé et le phosgène en excès sont refroidis dans un condenseur. A la fin de la réaction le milieu est refroidi, on fait passer un courant d'azote. Le chlorure d'acide chloré formé peut être isolé de façon habituelle, par exemple par distillation ou par cristallisation.

Le procédé selon l'invention permet à partir de matières premières disponibles sur le marché d'obtenir avec des rendements élevés des chlorures d'acides carboxyliques chlorés de grande pureté qui présentent de plus une excellente stabilité à la lumière et à la chaleur.

Les oxydes de phosphine utilisés comme catalyseurs sont très stables dans les conditions de la présente réaction et peuvent être utilisés une ou plusieurs fois avec la même efficacité. Il est donc possible de les recycler ou de réutiliser le résidu de distillation pour une nouvelle opération.

Les chlorures d'acides carboxyliques chlorés obtenus sont particulièrement utiles comme intermédiaires de synthèse par exemple pour la préparation des cétones chlorés ou pour former des produits pharmaceutiques tels que des neuroleptiques, des antiallergiques, des antidépresseurs et des antidiarrhéiques, ou des produits phytosanitaires tels que des insecticides.

Les exemples suivants illustrent l'invention :

### Exemple 1

Dans un réacteur de phosgénation, comportant un thermomètre, un agitateur, une arrivée de gaz et un condenseur, on introduit 172 g (2 moles) de 4-butyrolactone et 5,6 g (0,02 mole) d'oxyde de triphénylphosphine. On chauffe de mélange à 140°C et on fait barboter le phosgène gazeux.

Après avoir introduit 240 g de phosgène en 9 h de temps de réaction, de milieu réactionnel est refroidi et dégazé par bullage d'azote. Une distillation sous pression réduite permet de récupérer 233,5 g de chlorure de chloro-4-butyroyle, soit un rendement de 82% [point d'ébulltion : 82°C sous 44 mbar (33 mm Hg)]. La pureté de ce chlorure déterminée par analyse CPG est de 99%.

### Exemple 2

Dans un réacteur de 4 l, muni des mêmes dispositifs que précédemment, on introduit 2 500 g (29 moles) de 4-butyrolactone, 80 g (0,285 mode) d'oxyde de triphénylphosphine. Le milieu est chauffé à 140°C et de phosgène gazeux est introduit lentement. La transformation est complète au bout de 12h après avoir introduit 3 200 g de phosgène (32,3 moles).

Après dégazage et distillation sous pression réduite, on obtient 3 560 g de chlorure de chloro-4-butyroyle dont la pureté déterminée par analyse CPG est de 99%, soit un rendement de 86% [point d'ébullition 70°C sous 27 mbar (20 mm Hg)].

### Exemple 3

Dans un réacteur de 4 l, du même type que celui de l'exemple 2, on introduit 2 500 g (29 moles) de 4-butyrolactone, 500 g du pied de cuve de l'opération décrite à l'exemple 2 contenant d'équivalent de 80 g (0,285 mole) d'oxyde de triphénylphosphine. Le milieu est chauffé à 140°C et de phosgène gazeux est introduit lentement. La transformation est complète au bout de 13 h après avoir introduit 3 400 g de phosgène (34,34 moles). Après dégazage et distillation sous pression réduite, on obtient 3 640 g de chlorure de chloro-4-butyroyle (25,8 moles) de pureté 98,9% (analyse CPG), soit un rendement de 88,9% (point d'ébullition : 70°C sous 20 mm Hg).

Les résultats des tests de stabilité à la lumière et en enceinte climatique du chlorure obtenu sont des suivants :

| Stabilité à la lumière | | Coloration APHA | |
|---|---|---|---|
| Température | 20°C | Initiale | 30 |
| Durée | 1 mois | Final | 30 |

| Stabilité en enceinte climatique | | Coloration APHA | |
|---|---|---|---|
| Température | 50°C | Initiale | 30 |
| Durée | 1 mois | Final | 30 |

### Exemple 4

Comme à l'exemple 1, on introduit dans de réacteur 200 g (2 modes) de 4-valérolactone et 11,2 g (0,04 mole) d'oxyde de triphénylphosphine. On chauffe le milieu à 150°C et on introduit 268 g de phosgène gazeux en 9 h. Le milieu réactionnel est refroidi et dégaze. Le chlorure de chloro-4-méthyl-butyroyle est séparé par distillation [point d'ébullition : 68°C sous 24 mbar (18 mm Hg)]. Le rendement est de 70% par rapport à la 4-valérolactone ayant réagit.

### Exemple 5

Comme à l'exemple précédent, on introduit dans de réacteur 200 g (2 moles) de 5-valérolactone et 11,2 g (0,04 mole) d'oxyde de triphénylphosphine. On chauffe de mélange à 140°C. Après avoir introduit 236 g de phosgène gazeux en 5 h, le milieu réactionnel est refroidi et dégazé. Une distillation sous pression réduite permet de récupérer 217 g de chlorure de chloro-5-valéroyle, d'une pureté de 99% (analyse CPG) soit un rendement de 70% [point d'ébullition : 59°C sous 0.33 mbar (0,25 mm Hg)].

### Exemple 6

Comme précédemment, on introduit dans le réacteur 228,4 g (2 moles) de 6-caprolactone et 5,6 g (0,02 mole) d'oxyde de triphénylphosphine. On chauffe de mélange à 140°C et on introduit progressivement 265 g de phosgène gazeux. La conversion de la lactone est complète au bout de 6 h. Une distillation sous pression réduite permet de séparer 270 g de chlorure de chloro-6-caproyle, d'une pureté de 99%, soit un rendement de 79% (point d'ébullition : 67°C sous 0,3 mm Hg).

### Exemple 7

Comme précédemment, on introduit dans le réacteur 100 g (0, 419 mole) de 2,2-diphényl-4-butyrolactone et 1,15 g (4 x 10⁻³ mode) d'oxyde de triphénylphosphine. On chauffe progressivement le milieu à 150°C et on introduit lentement 72 g de phosgène gazeux à cette température. Après 15 h de reaction l'avancement de la réaction est de 60%. Le milieu est alors refroidi, puis dégazé à l'azote. Par distillation on obtient le chlorure de 2,2-diphényl-4-chloro-butyroyle. Point d'ébullition 200°C sous 1.2 mbar (0,9 mm Hg).

### Exemple 8

Dans un réacteur de phosgénation, comportant un thermomètre, un agitateur, une arrivée de gaz et un condenseur, on introduit 172 g (2 moles) de 4-butyrolactone et 5,9 g (0,02 mole) de sulfure de triphényl phosphine. Le mélange est porté à 140°C et on fait barboter le phosgène gazeux. Après avoir introduit 273 g de phosgène en 8 h de temps de réaction, de milieu réactionnel est refroidi et dégazé par bullage d'azote. Une distillation sous pression réduite permet de récupérer 200 g de chlorure de chloro-4 butyroyle (pureté 99% par analyse CPG), soit un rendement de 70%.

### Description pour les Etats contractants suivants : AT, SE

L'invention concerne un procédé de préparation de chlorures d'acides carboxyliques chlorés. Elle concerne plus particulièrement un procédé de préparation de chlorures d'acides carboxyliques aliphatiques chlorés par phosgénation de lactones aliphatiques.

Le brevet FR n° 1 080 261 décrit la préparation de chlorures d'acides carboxyliques chlorés par phosgénation de lactones en particulier de la 4-butyrolactone en présence de pyridine comme catalyseur à une température de 120°C. Mais il est difficile de reproduire les résultats mentionnés, en particulier ceux de l'exemple 2, comme il est indiqué dans la demande de brevet européen n° 253 214.

Dans l'article de D.J. Burton et W.M. Koppes (J. Org. Chem., Vol 40, N° 21, 1975, pp. 3026 à 3031), le chlorure aromatique d'orthochlorométhylbenzoyle a été obtenu à partir de la lactone aromatique correspondante avec un dichlorotriphénylphosphorane en excès mais ce composé est un réactif de laboratoire très cher qui n'est pas stable et est particulièrement sensible à l'hydrolyse. De plus il réagit avec le chlorure d'acide formé et il est préférable de l'utiliser après sa complexation avec un acide de Lewis tel que BF₃, ce qui rend encore plus compliqué le procédé. A part la transformation du phtalide mentionné aucun autre essai n'a été réalisé à partir d'autres lactones.

Selon la demande de brevet européen EP n° 253 214 récemment déposée on effectue la phosgénation de lactones telle que les butyrolactone, valérolactone ou caprolactone en présence d'un sel d'ammonium quaternaire à température élevée et de préférence en présence également d'acide chlorhydrique. Mais les sels d'ammonium quaternaires utilisés comme catalyseurs présentent l'inconvénient d'être plutôt instables à température élevée et perdent donc une partie de leur activité. L'acide chlorhydrique employé en quantité importante est particulièrement corrosif à haute température et nécessite des installations et des précautions particulières. Il se produit des réactions parasites. La lactone polymérise et l'agitation correcte du milieu n'est pas aisée. D'autres impuretés se forment par décomposition. La mise en oeuvre de ce procédé est par conséquent difficile. Plusieurs opérations successives sont nécessaires pour obtenir de bons rendements.

Les chlorures d'acides carboxyliques chlorés sont particulièrement recherchés depuis plusieurs années comme intermédiaires de synthèse pour la fabrication de médicaments et de phytosanitaires, il existe donc un besoin de les obtenir de façon simple et économique ainsi qu'avec de bons rendements. Ces chlorures doivent de plus présenter une bonne stabilité à la lumière et au stockage.

Il a maintenant été trouvé que l'on pouvait préparer des chlorures d'acides carboxyliques chlorés de formule 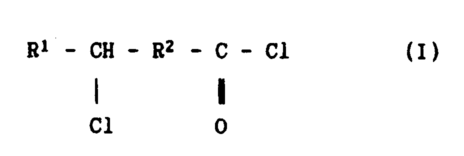 dans laquelle R² représente le radical (CH₂)n, n étant un nombre entier de 2 à 4, ou le radical -CH₂-C(C₆H₅)₂- et
R¹ représente un atome d'hydrogène ou un radical alkyle ayant de 1 à 4 atomes de carbone, par réaction des lactones de formule 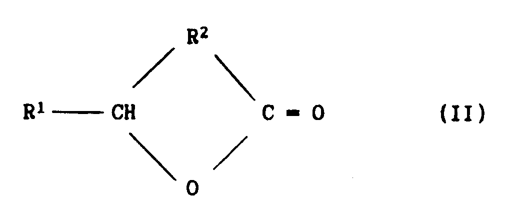 dans laquelle R¹ et R² ont les significations précédentes, avec du phosgène à une température comprise entre 90° et 180°C, en utilisant comme catalyseurs des oxydes ou des sulfures de phosphines trisubstitués de formule 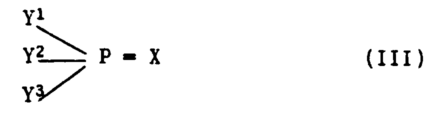 dans laquelle Y¹, Y² et Y³ identiques ou différents représentent chacun un radical alkyle linéaire ou ramifié ayant jusqu'à 18 atomes de carbone ou un radical phényle qui peut porter un ou plusieurs groupes alkyles ayant jusqu'à 4 atomes de carbone et X représente un atome d'oxygène ou dans laquelle Y¹, Y² et Y³ identiques ou différents représentent chacun un radical phényle qui peut porter un ou plusieurs groupes alkyles ayant jusqu'à 4 atomes de carbone et X représente un atome de soufre, ou les produits de réaction des composés de formule III avec des agents chlorurants, ou encore des mélanges de ces composés.

Les lactones qui peuvent être transformées selon le procédé selon l'invention sont de préférence les lactones de formule II dans laquelle R¹ représente un atome d'hydrogène ou un radical méthyle. A titre d'exemple on peut citer la 4-butyrolactone, la 4-valérolactone, la 5-valérolactone, la 6-caprolactone, la 2,2-diphényl-4-butyrolactone.

Des catalyseurs qui conviennent bien dans le cadre de la présente invention sont les composés de formule III dans lesquels Y¹, Y² et Y³ identiques ou différents représentent un radical alkyle linéaire ou ramifié ayant jusqu' à 12 atomes de carbone ou un radical phényle qui peut porter un ou plusieurs groupes méthyles.

On peut également remplacer les composés de formule III par leurs produits de réaction avec des agents chlorurants connus tels que le chlorure d'oxalyle, le chlorure de thionyle, le phosgène et le pentachlorure de phosphore, qui sont obtenus de façon classique par mélange des composés.

On peut également employer un mélange des composés de formule III et de ces produits de réaction.

Comme exemples de catalyseurs utilisables selon l'invention on peut citer l'oxyde de tributylphosphine, l'oxyde de trioctylphosphine, l'oxyde de tridodécylphosphine, l'oxyde de triphénylphosphine, le sulfure de triphénylphosphine, l'oxyde de tri-p-tolylphosphine.

Les catalyseurs préférés dans le cadre de la présente invention sont l'oxyde de trioctylphosphine et l'oxyde de triphénylphosphine.

Des quantités très faibles des catalyseurs précédemment décrits sont suffisantes pour la bonne réalisation du procédé. Elles sont généralement comprises entre 0,1 et 5% en mole par rapport à la lactone de départ et de préférence entre 0,5 et 2,5%.

La température de réaction peut être comprise entre 90° et 180°C. Elle est de préférence comprise entre 120° et 160°C.

La réaction est de préférence effectuée en absence de solvant. On peut cependant ajouter un milieu solvant inerte vis à vis des composés présents dans le milieu, en particulier du phosgène. Comme solvants on peut citer par exemple les solvants aromatiques chlorés ou non ayant un point d'ébullition suffisamment élevé tels que le monochlorobenzène, le dichlorobenzène, l'isopropylbenzène et les xylènes.

Le phosgène est généralement ajouté en quantité stoechiométrique ou en excès. Cet excès est de préférence compris entre 10 et 20%.

Le procédé selon la présente invention peut être mis en oeuvre soit en discontinu soit en continu à la pression atmosphérique ou à une pression voisine de la pression atmosphérique dans des dispositifs de phosgénation connus.

Selon un mode opératoire approprié, la lactone, le catalyseur et éventuellement le solvant sont tout d'abord introduits dans le réacteur. Le milieu réactionnel est chauffé à la température choisie puis on fait passer progressivement le phosgène gazeux dans le milieu. Le dioxyde de carbone formé et le phosgène en excès sont refroidis dans un condenseur. A la fin de la réaction le milieu est refroidi, on fait passer un courant d'azote. Le chlorure d'acide chloré formé peut être isolé de façon habituelle, par exemple par distillation ou par cristallisation.

Le procédé selon l'invention permet à partir de matières premières disponibles sur le marché d'obtenir avec des rendements élevés des chlorures d'acides carboxyliques chlorés de grande pureté qui présentent de plus une excellente stabilité à la lumière et à la chaleur.

Les oxydes de phosphine utilisés comme catalyseurs sont très stables dans les conditions de la présente réaction et peuvent être utilisés une ou plusieurs fois avec la même efficacité. Il est donc possible de les recycler ou de réutiliser le résidu de distillation pour une nouvelle opération.

Les chlorures d'acides carboxyliques chlorés obtenus sont particulièrement utiles comme intermédiaires de synthèse par exemple pour la préparation des cétones chlorés ou pour former des produits pharmaceutiques tels que des neuroleptiques, des antiallergiques, des antidépresseurs et des antidiarrhéiques, ou des produits phytosanitaires tels que des insecticides.

Les exemples suivants illustrent l'invention :

### Exemple 1

Dans un réacteur de phosgénation, comportant un thermomètre, un agitateur, une arrivée de gaz et un condenseur, on introduit 172 g (2 moles) de 4-butyrolactone et 5,6 g (0,02 mole) d'oxyde de triphénylphosphine. On chauffe de mélange à 140°C et on fait barboter le phosgène gazeux.

Après avoir introduit 240 g de phosgène en 9 h de temps de réaction, de milieu réactionnel est refroidi et dégazé par bullage d'azote. Une distillation sous pression réduite permet de récupérer 233,5 g de chlorure de chloro-4-butyroyle, soit un rendement de 82% [point d'ébulltion : 82°C sous 44 mbar (33 mm Hg)]. La pureté de ce chlorure déterminée par analyse CPG est de 99%.

### Exemple 2

Dans un réacteur de 4 l, muni des mêmes dispositifs que précédemment, on introduit 2 500 g (29 moles) de 4-butyrolactone, 80 g (0,285 mode) d'oxyde de triphénylphosphine. Le milieu est chauffé à 140°C et de phosgène gazeux est introduit lentement. La transformation est complète au bout de 12h après avoir introduit 3 200 g de phosgène (32,3 moles).

Après dégazage et distillation sous pression réduite, on obtient 3 560 g de chlorure de chloro-4-butyroyle dont la pureté déterminée par analyse CPG est de 99%, soit un rendement de 86% [point d'ébullition 70°C sous 27 mbar (20 mm Hg)].

### Exemple 3

Dans un réacteur de 4 l, du même type que celui de l'exemple 2, on introduit 2 500 g (29 moles) de 4-butyrolactone, 500 g du pied de cuve de l'opération décrite à l'exemple 2 contenant d'équivalent de 80 g (0,285 mole) d'oxyde de triphénylphosphine. Le milieu est chauffé à 140°C et de phosgène gazeux est introduit lentement. La transformation est complète au bout de 13 h après avoir introduit 3 400 g de phosgène (34,34 moles). Après dégazage et distillation sous pression réduite, on obtient 3 640 g de chlorure de chloro-4-butyroyle (25,8 moles) de pureté 98,9% (analyse CPG), soit un rendement de 88,9% (point d'ébullition : 70°C sous 20 mm Hg).

Les résultats des tests de stabilité à la lumière et en enceinte climatique du chlorure obtenu sont des suivants :

| Stabilité à la lumière | | Coloration APHA | |
|---|---|---|---|
| Température | 20°C | Initiale | 30 |
| Durée | 1 mois | Final | 30 |

| Stabilité en enceinte climatique | | Coloration APHA | |
|---|---|---|---|
| Température | 50°C | Initiale | 30 |
| Durée | 1 mois | Final | 30 |

### Exemple 4

Comme à d'exemple 1, on introduit dans de réacteur 172 g (2 modes) de 4-butyrolactone et 7,7 g (0,02 mole) d'oxyde de trioctylphosphine. On chauffe le mélange à 150°C et on introduit 272 g de phosgène gazeux en 15 h. Le milieu réactionnel est refroidi et dégazé. Une distillation sous pression réduite permet de récupérer 218,6 g de chlorure de chloro-4-butyroyle, pur à 98% (analyse CPG), soit un rendement de 76% [point d'ébullition 60°C sous 19 mbar (14 mm Hg)].

### Exemple 5

Comme à l'exemple 1, on introduit dans de réacteur 200 g (2 modes) de 4-valérolactone et 11,2 g (0,04 mole) d'oxyde de triphénylphosphine. On chauffe le milieu à 150°C et on introduit 268 g de phosgène gazeux en 9 h. Le milieu réactionnel est refroidi et dégazé. Le chlorure de chloro-4-méthyl-butyroyle est séparé par distillation [point d'ébullition : 68°C sous 24 mbar (18 mm Hg)]. Le rendement est de 70% par rapport à la 4-valérolactone ayant réagit.

### Exemple 6

Comme à l'exemple précédent, on introduit dans de réacteur 200 g (2 moles) de 5-valérolactone et 11,2 g (0,04 mole) d'oxyde de triphénylphosphine. On chauffe de mélange à 140°C. Après avoir introduit 236 g de phosgène gazeux en 5 h, le milieu réactionnel est refroidi et dégazé. Une distillation sous pression réduite permet de récupérer 217 g de chlorure de chloro-5-valéroyle, d'une pureté de 99% (analyse CPG) soit un rendement de 70% [point d'ébullition : 59°C sous 0.33 mbar (0,25 mm Hg)].

### Exemple 7

Comme précédemment, on introduit dans le réacteur 228,4 g (2 moles) de 6-caprolactone et 5,6 g (0,02 mole) d'oxyde de triphénylphosphine. On chauffe de mélange à 140°C et on introduit progressivement 265 g de phosgène gazeux. La conversion de la lactone est complète au bout de 6 h. Une distillation sous pression réduite permet de séparer 270 g de chlorure de chloro-6-caproyle, d'une pureté de 99%, soit un rendement de 79% (point d'ébullition : 67°C sous 0,3 mm Hg).

### Exemple 8

Comme précédemment, on introduit dans le réacteur 100 g (0, 419 mole) de 2,2-diphényl-4-butyrolactone et 1,15 g (4 x 10⁻³ mode) d'oxyde de triphénylphosphine. On chauffe progressivement le milieu à 150°C et on introduit lentement 72 g de phosgène gazeux à cette température. Après 15 h de réaction l'avancement de la réaction est de 60%. Le milieu est alors refroidi, puis dégazé à l'azote. Par distillation on obtient le chlorure de 2,2-diphényl-4-chloro-butyroyle. Point d'ébullition 200°C sous 1.2 mbar (0,9 mm Hg).

### Exemple 9

Dans un réacteur de phosgénation, comportant un thermomètre, un agitateur, une arrivée de gaz et un condenseur, on introduit 172 g (2 moles) de 4-butyrolactone et 5,9 g (0,02 mole) de sulfure de triphényl phosphine. Le mélange est porté à 140°C et on fait barboter le phosgène gazeux. Après avoir introduit 273 g de phosgène en 8 h de temps de réaction, de milieu réactionnel est refroidi et dégazé par bullage d'azote. Une distillation sous pression réduite permet de récupérer 200 g de chlorure de chloro-4 butyroyle (pureté 99% par analyse CPG), soit un rendement de 70%.

### Exemple 10

De la même façon qu'à l'exemple précédent, on introduit dans le réacteur 172 g (2 moles) de 4-butyrolactone et 4,4 g (0,02 mode) d'oxyde de tributyl phosphine. On chauffe à 150°C et on fait barboter le phosgène gazeux. Après avoir introduit 280 g de phosgène en 15 heures de réaction, le milieu réactionnel est refroidi et dégazé par budlage d'azote. Après distillation sous pression réduire, on recueille 171 g de chlorure de chloro-4 butyroyle (pureté 99% par analyse CPG), soit un rendement de 60%.

## Revendications (Revendications pour l'(les) Etat(s) contractant(s) suivant(s): BE, CH, DE, ES, FR, GB, IT, LI)

1. Procédé de préparation des chlorures d'acides carboxyliques chlorés de formule 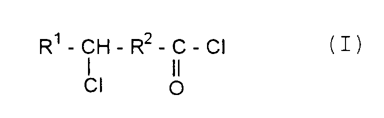 dans laquelle R² représente le radical (CH₂)ₙ, n étant un nombre entier de 2 à 4 ou le radical -CH₂-C(C₆H₅)₂- et
R¹ représente un atome d'hydrogène ou un radical alkyle ayant de 1 à 4 atomes de carbone, par réaction d'une lactone de formule 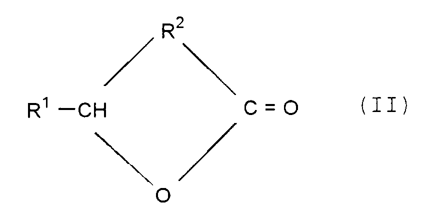 dans laquelle R¹ et R² ont les significations précédentes, avec du phosgène en présence d'un catalyseur, caractérisé en ce que la réaction est effectuée à une température de 90° à 180°C, en utilisant comme catalyseurs les composés choisis dans le groupe constitué par les oxydes ou les sulfures de phosphines trisubstitués de formule 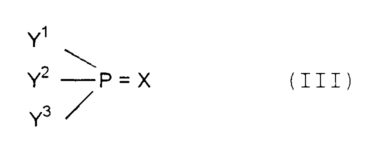 dans laquelle Y¹, Y² et Y³, identiques ou différents, représentent chacun un radical phényle qui peut porter un ou plusieurs groupes alkyles ayant jusqu'à 4 atomes de carbone et X représente un atome d'oxygène ou de soufre; les produits de réaction des composés de formule (III) avec des agents chlorurants; et les mélanges de ces composés.

2. Procédé de préparation des chlorures d'acides carboxyliques chlorés de formule 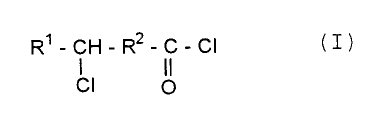 dans laquelle R² représente le radical -CH₂-C(C₆H₅)₂- et
R¹ représente un atome d'hydrogène ou un radical alkyle ayant de 1 à 4 atomes de carbone, par réaction d'une lactone de formule 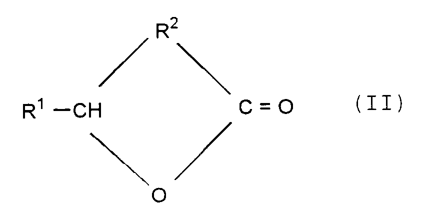 dans laquelle R¹ et R² ont les significations précédentes, avec du phosgène en présence d'un catalyseur, caractérisé en ce que la réaction est effectuée à une température de 90° à 180°C, en utilisant comme catalyseurs les composés choisis dans le groupe constitué par les oxydes de phosphines trisubstitués de formule 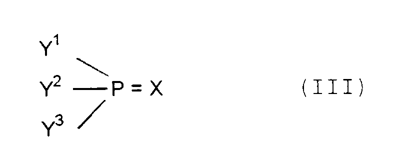 dans laquelle Y¹, Y² et Y³, identiques ou différents, représentent chacun un radical alkyle linéaire ou ramifié ayant jusqu'à 18 atomes de carbone et X représente un atome d'oxygène; les produits de réaction des composés de formule (III) avec des agents chlorurants; et les mélanges de ces composés.

3. Procédé selon la revendication 1, caractérisé en ce que Y¹, Y² et Y³, identiques ou différents, représentent chacun un radical phényle qui peut porter un ou plusieurs groupes méthyles.

4. Procédé selon l'une quelconque des revendications précédentes, caractérisé en ce que le catalyseur est ajouté en proportions comprises entre 0,1 et 5%, de préférence entre 0,5 et 2,5% en mole par rapport à la lactone.

5. Procédé selon l'une quelconque des revendications précédentes, caractérisé en ce que le phosgène est ajouté en quantité stoechiométrique ou en excès, de préférence compris entre 10 et 20% en mole par rapport à la lactone.

6. Procédé selon l'une quelconque des revendications 1 et 3 à 5, caractérisé en ce que le catalyseur utilisé est l'oxyde de triphénylphosphine.

7. Procédé selon l'une quelconque des revendications précédentes, caractérisé en ce que la température est comprise entre 120° et 160°C.

8. Procédé selon l'une quelconque des revendications précédentes, caractérisé en ce que la réaction a lieu dans un milieu solvant inerte vis à vis des composés présents choisis parmi les hydrocarbures aromatiques chlorés ou non.

9. Procédé selon l'une quelconque des revendications précédentes, caractérisé en ce que R¹ représente un atome d'hydrogène ou un radical méthyle.

10. Procédé selon l'une quelconque des revendications 1 et 3 à 9, caractérisé en ce que la lactone est choisie parmi la 4-butyrolactone, la 4-valérolactone, la 5-valérolactone, la 6-caprolactone, la 2,2-diphényl-4-butyrolactone.

## Revendications (Revendications pour l'(les) Etat(s) contractant(s) suivant(s): AT, SE)

1. Procédé de préparation des chlorures d'acides carboxyliques chlorés de formule 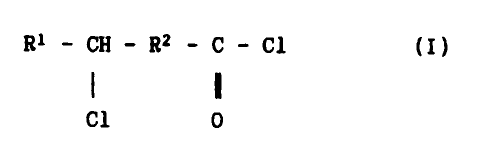 dans laquelle R² représente de radical (CH₂)n, n étant un nombre entier de 2 à 4, ou le radical -CH₂-C(C₆H₅)₂- et
R¹ représente un atome d'hydrogène ou un radical alkyle ayant de 1 à 4 atomes de carbone, par réaction des lactones de formule 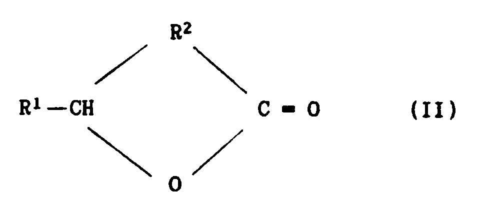 dans laquelle R¹ et R² ont des significations précédentes, avec du phosgène, caractérisé en ce que la réaction est effectuée à une température de 90° à 180 °C en utilisant comme catalyseurs des oxydes ou sulfures de phosphines trisubstitués de formule 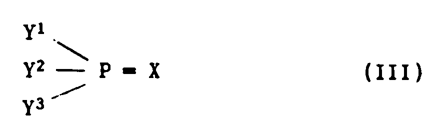 dans laquelle Y¹, Y² et Y³ identiques ou différents représentent chacun un radical alkyle linéaire ou ramifié ayant jusqu'à 18 atomes de carbone ou un radical phényle qui peut porter un ou plusieurs groupes alkyles ayant jusqu'à 4 atomes de carbone et X représente un atome d'oxygène ou dans laquelle Y¹, Y² et Y³ identiques ou différents représentent chacun un radical phényle qui peut porter un ou plusieurs groupes alkyles ayant jusqu'à 4 atomes de carbone et X représente un atome de soufre, ou des produits de réaction des composés de formule III avec des agents chlorurants, ou encore des mélanges de ces composés.

2. Procédé selon la revendication 1 caractérisé en ce que le catalyseur est ajouté en proportions comprises entre 0,1 et 5%, de préférence entre 0,5 et 2,5% en mode par rapport à la lactone.

3. Procédé selon l'une quelconque des revendications précédentes caractérisé en ce que Y¹, Y² et Y³ identiques ou différents représentent chacun un radical alkyle linéaire ou ramifié ayant jusqu'à 12 atomes de carbone ou un radical phényle qui peut porter un ou plusieurs groupes méthyles.

4. Procédé selon l'une quelconque des revendications précédentes caractérisé en ce que le phosgène est ajouté en quantité stoechiométrique ou en excès, de préférence compris entre 10 et 20% en mole par rapport à la lactone.

5. Procédé selon l'une quelconque des revendications précédentes caractérisé en ce que le catalyseur utilisé est l'oxyde de trioctylphosphine ou l'oxyde de triphénylphosphine.

6. Procédé selon l'une quelconque des revendications précédentes caractérisé en ce que la température est comprise entre 120° et 160°C.

7. Procédé selon l'une quelconque des revendications précédentes caractérisé en ce que la réaction a lieu dans un milieu solvant inerte vis à vis des composés présents choisis parmi les hydrocarbures aromatiques chlorés ou non.

8. Procédé selon l'une quelconque des revendications précédentes caractérisé en ce que R¹ représente un atome d'hydrogène ou un radical méthyle.

9. Procédé selon l'une quelconque des revendications précédentes caractérisé en ce que la lactone est choisie parmi la 4-butyrolactone, la 4-valérolactone, la 5-valérolactone, la 6-caprolactone, la 2, 2-diphényl-4-butyrolactone.

## Claims (Claims for the following Contracting State(s): BE, CH, DE, ES, FR, GB, IT, LI)

1. Process for preparing chlorinated carboxylic acid chlorides of formula 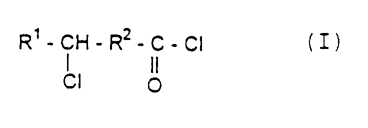 in which R² represents the (CH₂)ₙ radical, n being an integer from 2 to 4, or the radical -CH₂-C(C₆H₅)₂- and
R¹ represents a hydrogen atom or an alkyl radical having 1 to 4 carbon atoms, by reacting a lactone of formula 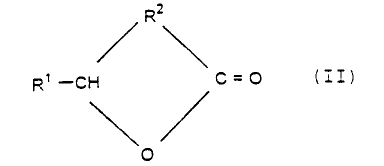 in which R¹ and R² have the meanings above, with phosgene in the presence of a catalyst, characterized in that the reaction is carried out at a temperature of 90° to 180°C, using as catalysts the compounds chosen from the group composed of trisubstituted oxides or sulphides of phosphines of formula 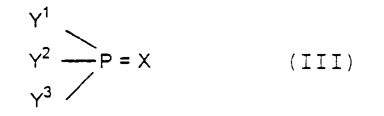 in which Y¹, Y² and Y³, which are identical or different, each represent a phenyl radical which may carry one or more alkyl groups having up to 4 carbon atoms and X represents an oxygen or sulphur atom; the products of the reaction of the compounds of formula (III) with chlorinating agents; and mixtures of these compounds.

2. Process for preparing chlorinated carboxylic acid chlorides of formula 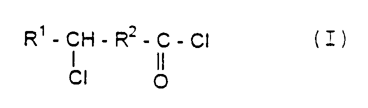 in which R² represents the radical -CH₂-C(C₆H₅)₂- and
R¹ represents a hydrogen atom or an alkyl radical having from 1 to 4 carbon atoms, by reacting a lactone of formula 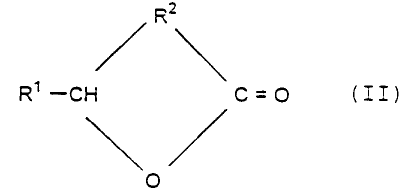 in which R¹ and R² have the meanings above, with phosgene in the presence of a catalyst, characterized in that the reaction is carried out at a temperature of 90° to 180°C, using as catalysts the compounds chosen from the group composed of trisubstituted oxides of phosphines of formula 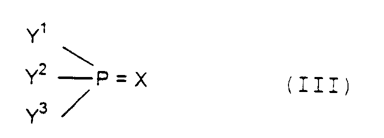 in which Y¹, Y² and Y³, which are identical or different, each represent a linear or branched alkyl radical having up to 18 carbon atoms and X represents an oxygen atom; the products of the reaction of the compounds of formula (III) with chlorinating agents; and mixtures of these compounds.

3. Process according to Claim 1, characterized in that Y¹, Y² and Y³, which are identical or different, each represent a phenyl radical which may carry one or more methyl groups.

4. Process according to any one of the preceding claims, characterized in that the catalyst is added in proportions of between 0.1 and 5 mol %, preferably between 0.5 and 2.5 mol % relative to the lactone.

5. Process according to any one of the preceding claims, characterized in that the phosgene is added in a stoichiometric quantity or in excess, preferably between 10 and 20 mol % relative to the lactone.

6. Process according to any one of Claims 1 and 3 to 5, characterized in that the catalyst used is triphenylphosphine oxide.

7. Process according to any one of the preceding claims, characterized in that the temperature is between 120° and 160°C.

8. Process according to any one of the preceding claims, characterized in that the reaction occurs in a solvent medium which is inert in relation to the compounds present chosen from aromatic hydrocarbons, chlorinated or otherwise.

9. Process according to any one of the preceding claims, characterized in that R¹ represents a hydrogen atom or a methyl radical.

10. Process according to any one of Claims 1 and 3 to 9, characterized in that the lactone is chosen from 4-butyrolactone, 4-valerolactone, 5-valerolactone, 6-caprolactone, 2,2-diphenyl-4-butyrolactone.

## Claims (Claims for the following Contracting State(s): AT, SE)

1. Process for preparing chlorinated carboxylic acid chlorides of formula 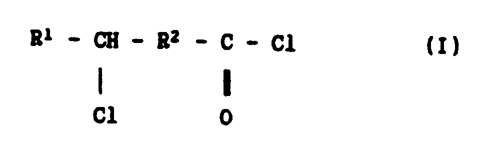 in which R² represents the (CH₂)ₙ radical, n being an integer from 2 to 4, or the radical -CH₂-C(C₆CH₅)₂- and
R¹ represents a hydrogen atom or an alkyl radical having 1 to 4 carbon atoms, by reacting the lactones of formula 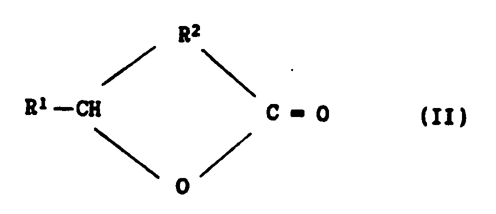 in which R¹ and R² have the meanings above, with phosgene, characterized in that the reaction is carried out at a temperature of 90° to 180°C, using as catalysts trisubstituted oxides or sulphides of phosphines of formula 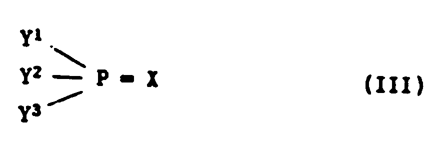 in which Y¹, Y² and Y³, which are identical or different, each represent a linear or branched alkyl radical having up to 18 carbon atoms or a phenyl radical which may carry one or more alkyl groups having up to 4 carbon atoms and X represents an oxygen atom, or in which Y¹, Y² and Y³, which are identical or different, each represent a phenyl radical which may carry one or more alkyl groups having up to 4 carbon atoms and X represents a sulphur atom, or the products of the reaction of the compounds of formula III with chlorinating agents, or alternatively mixtures of these compounds.

2. Process according to Claim 1, characterized in that the catalyst is added in proportions of between 0.1 and 5 mol %, preferably between 0.5 and 2.5 mol % relative to the lactone.

3. Process according to any one of the preceding claims, characterized in that Y¹, Y² and Y³, which are identical or different, each represent a linear or branched alkyl radical having up to 12 carbon atoms or a phenyl radical which may carry one or more methyl groups.

4. Process according to any one of the preceding claims, characterized in that the phosgene is added in a stoichiometric quantity or in excess, preferably between 10 and 20 mol % relative to the lactone.

5. Process according to any one of the preceding claims, characterized in that the catalyst used is trioctylphosphine oxide or triphenylphosphine oxide.

6. Process according to any one of the preceding claims, characterized in that the temperature is between 120° and 160°C.

7. Process according to any one of the preceding claims, characterized in that the reaction occurs in a solvent medium which is inert in relation to the compounds present chosen from aromatic hydrocarbons, chlorinated or otherwise.

8. Process according to any one of the preceding claims, characterized in that R¹ represents a hydrogen atom or a methyl radical.

9. Process according to any one of the preceding claims, characterized in that the lactone is chosen from 4-butyrolactone, 4-valerolactone, 5-valerolactone, 6-caprolactone, 2,2-diphenyl-4-butyrolactone.

## Patentansprüche (Patentansprüche für folgende(n) Vertragsstaat(en): BE, CH, DE, ES, FR, GB, IT, LI)

1. Verfahren zur Herstellung von Chloriden chlorierter Carbonsäuren der Formel 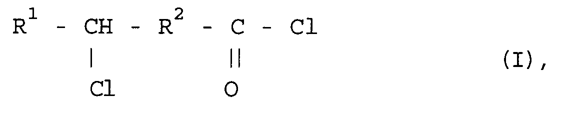 in der bedeuten:
R¹ ein Wasserstoffatom oder C₁₋₄-Alkyl
und
R² eine Gruppe (CH₂)ₙ, wobei n eine ganze Zahl von 2 bis 4 bedeutet, oder die Gruppe -CH₂-C(C₆H₅)₂-,
durch Umsetzung von Lactonen der Formel 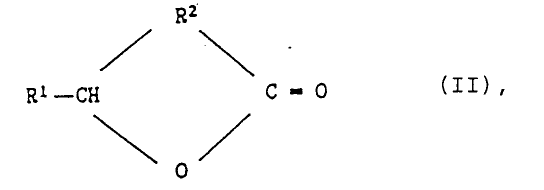 in der R¹ und R² die oben angegebenen Bedeutungen haben,
mit Phosgen in Gegenwart eines Katalysators,
dadurch gekennzeichnet, daß die Umsetzung bei einer Temperatur von 90 bis 180 °C durchgeführt wird, wobei als Katalysatoren verwendet werden:
- Oxide oder Sulfide von trisubstituierten Phosphinen der Formel 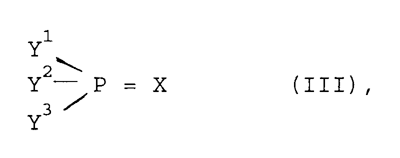 worin bedeuten:
Y¹, Y² und Y³, die gleich oder verschieden sind, jeweils eine Phenylgruppe, die ggf. mit einer oder mehreren C₁₋₄-Alkylgruppen substituiert ist,
und
X ein Sauerstoffatom oder ein Schwefelatom,
- Reaktionsprodukte von Verbindungen der Formel III mit Chlorierungsmitteln
oder
- Gemische dieser Verbindungen.

2. Verfahren zur Herstellung von Chloriden chlorierter Carbonsäuren der Formel 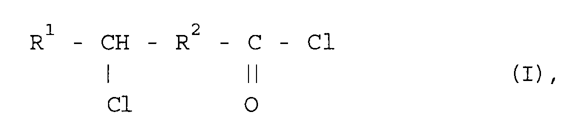 in der bedeuten:
R¹ ein Wasserstoffatom oder C₁₋₄-Alkyl
und
R² die Gruppe -CH₂-C(C₆H₅)₂-,
durch Umsetzung von Lactonen der Formel 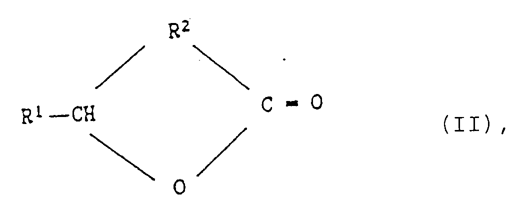 in der R¹ und R² die oben angegebenen Bedeutungen haben,
mit Phosgen in Gegenwart eines Katalysators,
dadurch gekennzeichnet, daß die Umsetzung bei einer Temperatur von 90 bis 180 °C durchgeführt wird, wobei als Katalysatoren
- Oxide von trisubstituierten Phosphinen der Formel 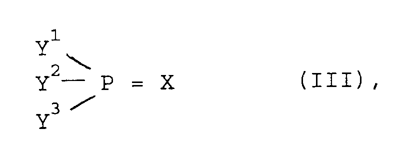 worin bedeuten:
Y¹, Y² und Y³, die gleich oder verschieden sind, jeweils geradkettiges oder verzweigtes Alkyl mit bis zu 18 C-Atomen
und
X ein Sauerstoffatom,
oder
- Reaktionsprodukte von Verbindungen der Formel III mit Chlorierungsmitteln
oder
- Gemische dieser Verbindungen
verwendet werden.

3. Verfahren nach Anspruch 1, dadurch gekennzeichnet, daß Y¹, Y² und Y³, die gleich oder verschieden sind, jeweils eine Phenylgruppe bedeuten, die ggf. mit einer oder mehreren Methylgruppen substituiert ist.

4. Verfahren nach einem der vorhergehenden Ansprüche, dadurch gekennzeichnet, daß der Katalysator in Mengenanteilen von 0,1 bis 5 Mol-% und vorzugsweise 0,5 bis 2,5 Mol-%, bezogen auf das Lacton, zugegeben wird.

5. Verfahren nach einem der vorhergehenden Ansprüche, dadurch gekennzeichnet, daß das Phosgen in stöchiometrischer Menge oder im Überschuß, vorzugsweise in einem Überschuß von 10 bis 20 Mol-%, bezogen auf das Lacton, zugegeben wird.

6. Verfahren nach einem der Ansprüche 1 und 3 bis 5, dadurch gekennzeichnet, daß als Katalysator Triphenylphosphinoxid verwendet wird.

7. Verfahren nach einem der vorhergehenden Ansprüche, dadurch gekennzeichnet, daß die Temperatur im Bereich von 120 bis 160 °C liegt.

8. Verfahren nach einem der vorhergehenden Ansprüche, dadurch gekennzeichnet, daß die Umsetzung in einem gegenüber den vorliegenden Verbindungen inerten Lösungsmittelmedium durchgeführt wird, wobei die Lösungsmittel unter aromatischen Kohlenwasserstoffen und chlorierten aromatischen Kohlenwasserstoffen ausgewählt sind.

9. Verfahren nach einem der vorhergehenden Ansprüche, dadurch gekennzeichnet, daß R¹ ein Wasserstoffatom oder Methyl bedeutet.

10. Verfahren nach einem der vorhergehenden Ansprüche, dadurch gekennzeichnet, daß das Lacton unter 4-Butyrolacton, 4-Valerolacton, 5-Valerolacton, 6-Caprolacton und 2,2-Diphenyl-4-butyrolacton ausgewählt wird.

## Patentansprüche (Patentansprüche für folgende(n) Vertragsstaat(en): AT, SE)

1. Verfahren zur Herstellung von Chloriden chlorierter Carbonsäuren der Formel 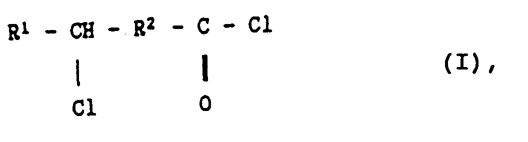 in der bedeuten:
R¹ ein Wasserstoffatom oder C₁₋₄-Alkyl
und
R² eine Gruppe (CH₂)ₙ, wobei n eine ganze Zahl von 2 bis 4 bedeutet, oder die Gruppe -CH₂-C(C₆H₅)₂-
durch Umsetzung von Lactonen der Formel 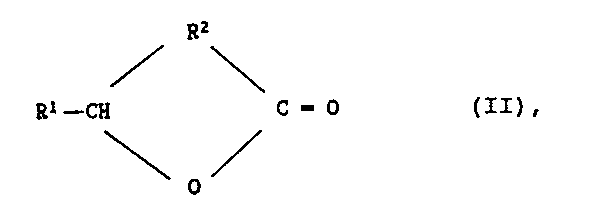 in der R¹ und R² die oben angegebenen Bedeutungen haben,
mit Phosgen,
dadurch gekennzeichnet, daß die Umsetzung bei einer Temperatur von 90 bis 180 ° C durchgeführt wird, wobei als Katalysatoren
- Oxide oder Sulfide von trisubstituierten Phosphinen der Formel 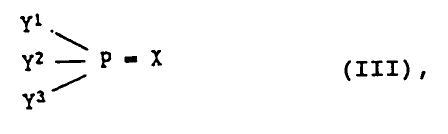 worin bedeuten:
Y¹, Y² und Y³, die gleich oder verschieden sind, jeweils geradkettiges oder verzweigtes C₁₋₁₈-Alkyl oder eine Phenylgruppe, die ggfs. mit einer oder mehreren C₁₋₄-Alkylgruppen substituiert ist,
und
X ein Sauerstoffatom
oder
Y¹, Y² und Y³, die gleich oder verschieden sind, jeweils Phenyl, das ggfs. mit einer oder mehreren C₁₋₄-Alkylgruppen substituiert ist,
und
X ein Schwefelatom,
oder
- Reaktionsprodukte von Verbindungen der Formel III mit Chlorierungsmitteln oder
- Gemische dieser Verbindungen
verwendet werden.

2. Verfahren nach Anspruch 1, dadurch gekennzeichnet, daß der Katalysator in Mengenanteilen von 0,1 bis 5 Mol-% und vorzugsweise 0,5 bis 2,5 Mol-%, bezogen auf das Lacton, zugegeben wird.

3. Verfahren nach Anspruch 1 oder 2, dadurch gekennzeichnet, daß Y¹, Y² und Y³, die gleich oder verschieden sind, jeweils geradkettiges oder verzweigtes C₁₋₁₂-Alkyl oder eine Phenylgruppe bedeuten, die ggfs. mit einer oder mehreren Methylgruppen substituiert ist.

4. Verfahren nach einem der vorhergehenden Ansprüche, dadurch gekennzeichnet, daß das Phosgen in stöchiometrischer Menge oder im Überschuß, vorzugsweise von 10 bis 20 Mol-%, bezogen auf das Lacton, eingesetzt wird.

5. Verfahren nach einem der vorhergehenden Ansprüche, dadurch gekennzeichnet, daß als Katalysator Trioctylphosphinoxid oder Triphenylphosphinoxid verwendet wird.

6. Verfahren nach einem der vorhergehenden Ansprüche, dadurch gekennzeichnet, daß die Temperatur im Bereich von 120 bis 160 °C liegt.

7. Verfahren nach einem der vorhergehenden Ansprüche, dadurch gekennzeichnet, daß die Umsetzung in einem gegenüber den vorliegenden Verbindungen inerten Lösungsmittelmedium durchgeführt wird, wobei die Lösungsmittel unter aromatischen Kohlenwasserstoffen und chlorierten aromatischen Kohlenwasserstoffen ausgewählt sind.

8. Verfahren nach einem der vorhergehenden Ansprüche, dadurch gekennzeichnet, daß R¹ ein Wasserstoffatom oder Methyl bedeutet.

9. Verfahren nach einem der vorhergehenden Ansprüche, dadurch gekennzeichnet, daß das Lacton unter 4-Butyrolacton, 4-Valerolacton, 5-Valerolacton, 6-Caprolacton und 2,2-Diphenyl-4-butyrolacton ausgewählt wird.
